# EUROPEAN PATENT APPLICATION

(11) **EP 4 803 004 A2**
(43) Date of publication of application: **09.09.2026**
(21) Application number: 26193352.7
(22) Date of filing: 30.08.2022
(51) Int. Cl.: A61B 5/383

(54) **LOW POWER FEEDBACK-CONTROLLED NEURAL STIMULATION SYSTEM**

(30) Priority: 30.08.2021 AU 2021902812
(62) Document number(s) of the earlier application(s) in accordance with Art. 76 EPC: 22862420.1 / 4 395 877
(71) Applicant: Saluda Medical Pty Ltd, Macquarie Park NSW 2113 (AU)
(72) Inventor: SINGLE, Peter Scott Vallack, Artarmon, 2064 (AU)
(74) Representative: Abel & Imray LLP

(57) **Abstract**

Disclosed is an implantable neural stimulation device, the device comprising: an electrode array comprising a plurality of electrodes, the electrodes comprising a first stimulus electrode and a second stimulus electrode; a pulse generator connectable to the stimulus electrodes, the pulse generator configured to generate a multiphasic stimulus pulse of current from a supply voltage and deliver the multiphasic stimulus pulse via the stimulus electrodes to an electrically excitable tissue in order to evoke a neural response on a neural pathway in the electrically excitable tissue; and modulation circuitry connectable to a regulation electrode of the plurality of electrodes, the modulation circuitry configured to modulate a voltage on the regulation electrode during the delivery of the multiphasic stimulus pulse such that a corresponding voltage on each stimulus electrode varies substantially symmetrically around a value which is about half the supply voltage over the multiphasic stimulus pulse.

## Description

### TECHNICAL FIELD

The present invention relates to feedback-controlled neural stimulation for pain management, and in particular to reducing the power consumption of neural stimulation while minimising stimulation artefact in the measurement of neural response.

### BACKGROUND OF THE INVENTION

There are a range of situations in which it is desirable to apply neural stimuli in order to alter neural function, a process known as neuromodulation. For example, neuromodulation is used to treat a variety of disorders including chronic neuropathic pain, Parkinson's disease, and migraine. A neuromodulation system applies an electrical pulse (stimulus) to neural tissue (fibres, or neurons) in order to generate a therapeutic effect. In general, the electrical stimulus generated by a neuromodulation system evokes a neural response known as an action potential in a neural fibre which then has either an inhibitory or excitatory effect. Inhibitory effects can be used to modulate an undesired process such as the transmission of pain, or excitatory effects may be used to cause a desired effect such as the contraction of a muscle.

When used to relieve neuropathic pain originating in the trunk and limbs, the electrical pulse is applied to the dorsal column (DC) of the spinal cord, a procedure referred to as spinal cord stimulation (SCS). Such a system typically comprises an implanted electrical pulse generator, and a power source such as a battery that may be transcutaneously rechargeable by wireless means, such as inductive transfer. An electrode array is connected to the pulse generator, and is positioned adjacent the target neural fibre(s) in the spinal cord, typically in the dorsal epidural space above the dorsal column. An electrical pulse of sufficient intensity applied to the target neural fibres by a stimulus electrode causes the depolarisation of neurons in the fibres, which in turn generates an action potential in the fibres. Action potentials propagate along the fibres in orthodromic (towards the head, or rostral) and antidromic (towards the cauda, or caudal) directions. The fibres being stimulated in this way inhibit the transmission of pain from a region of the body innervated by the target neural fibres (the dermatome) to the brain. To sustain the pain relief effects, stimuli are applied repeatedly, for example periodically, at a frequency in the range of 30 Hz - 100 Hz.

For effective and comfortable neuromodulation, it is necessary to maintain stimulus intensity above a recruitment threshold. Stimuli below the recruitment threshold will fail to recruit sufficient neurons to generate action potentials with a therapeutic effect. In almost all neuromodulation applications, response from a single class of fibre is desired, but the stimulus waveforms employed can evoke action potentials in other classes of fibres which cause unwanted side effects. In pain relief, is therefore necessary to apply stimuli with intensity below a comfort threshold, above which uncomfortable or painful percepts arise due to over-recruitment of Aβ (A-beta) fibres. When recruitment is too large, Aβ fibres produce uncomfortable sensations. Stimulation at high intensity may even recruit Aδ (A-delta) fibres, which are sensory nerve fibres associated with acute pain, cold and pressure sensation. It is therefore desirable to maintain stimulus intensity within a therapeutic range between the recruitment threshold and the comfort threshold.

The task of maintaining appropriate neural recruitment is made more difficult by electrode migration (change in position over time) and/or postural changes of the implant recipient (patient), either of which can significantly alter the neural recruitment arising from a given stimulus, and therefore the therapeutic range. There is room in the epidural space for the electrode array to move, and such array movement from migration or posture change alters the electrode-to-fibre distance and thus the recruitment efficacy of a given stimulus. Moreover, the spinal cord itself can move within the cerebrospinal fluid (CSF) with respect to the dura. During postural changes, the amount of CSF and/or the distance between the spinal cord and the electrode can change significantly. This effect is so large that postural changes alone can cause a previously comfortable and effective stimulus regime to become either ineffectual or painful.

Another control problem facing neuromodulation systems of all types is achieving neural recruitment at a sufficient level for therapeutic effect, but at minimal expenditure of energy. The power consumption of the stimulation paradigm has a direct effect on battery requirements which in turn affects the device's physical size and lifetime. For rechargeable systems, increased power consumption results in more frequent charging and, given that batteries only permit a limited number of charging cycles, ultimately this reduces the implanted lifetime of the device.

Attempts have been made to address such problems by way of feedback or closed-loop control, such as using the methods set forth in International Patent Publication No. WO2012/155188 by the present applicant. Feedback control seeks to compensate for relative nerve / electrode movement by controlling the intensity of the delivered stimuli so as to maintain a substantially constant neural recruitment. The intensity of a neural response evoked by a stimulus may be used as a feedback variable representative of the amount of neural recruitment. A signal representative of the neural response may be generated by a measurement electrode in electrical communication with the recruited neural fibres, and processed to obtain the feedback variable. Based on the response intensity, the intensity of the applied stimulus may be adjusted to maintain the response intensity within a therapeutic range.

It is therefore desirable to accurately detect and record a neural response evoked by the stimulus. The action potentials generated by the depolarisation of a large number of fibres by a stimulus sum to form a measurable signal known as an evoked compound action potential (ECAP). Accordingly, an ECAP is the sum of responses from a large number of single fibre action potentials. The ECAP generated from the depolarisation of a group of similar fibres may be measured at a measurement electrode as a positive peak potential, then a negative peak, followed by a second positive peak. This morphology is caused by the region of activation passing the measurement electrode as the action potentials propagate along the individual fibres.

Approaches proposed for obtaining a neural response measurement are described by the present applicant in International Patent Publication No. WO 2012/155183, the content of which is incorporated herein by reference.

However, neural response measurement can be a difficult task as an observed CAP signal component in the measured response will typically have a maximum amplitude in the range of microvolts. In contrast, a stimulus applied to evoke the CAP is typically several volts, and manifests in the measured response as crosstalk of that magnitude. Moreover, stimulus generally results in electrode artefact, which manifests in the measured response as a decaying output of the order of several millivolts after the end of the stimulus. As the CAP signal can be contemporaneous with the stimulus crosstalk and/or the stimulus artefact, CAP measurements present a difficult challenge of measurement amplifier design. For example, to resolve a 10 µV CAP with 1 µV resolution in the presence of stimulus crosstalk of 5 V requires an amplifier with a dynamic range of 134 dB, which is impractical in implantable devices. In practice, many non-ideal aspects of a circuit lead to artefact, and as these aspects mostly result a time-decaying artefact waveform of positive or negative polarity, their identification and elimination can be laborious.

Evoked neural responses are less difficult to detect when they appear later in time than the artefact, or when the signal-to-noise ratio is sufficiently high. The artefact is often restricted to a time of 1 - 2 ms after the stimulus and so, provided the neural response is detected after this time window, a neural response measurement can be more easily obtained. This is the case in surgical monitoring where there are large distances (e.g. more than 12 cm for nerves conducting at 60 ms⁻¹) between the stimulating and measurement electrodes so that the propagation time from the stimulus site to the measurement electrodes exceeds 2 ms.

However, to characterize the responses from the dorsal column, high stimulation currents are required. Similarly, any implanted neuromodulation device will necessarily be of compact size, so that for such devices to monitor the effect of applied stimuli, the stimulus electrode(s) and measurement electrode(s) will necessarily be in close proximity. In such situations the measurement process should overcome artefact directly.

The difficulty of this problem is further exacerbated when attempting to implement CAP detection in an implanted device. Typical implanted devices have a power budget that permits a limited number, for example in the hundreds or low thousands, of processor instructions per stimulus, in order to maintain a desired battery lifetime. Accordingly, if a CAP detector for an implanted device is to be used regularly (e.g. once a second), then care must be taken that the detector should consume only a small fraction of the power budget.

Therefore, a need exists for a neuromodulation device that delivers stimulus and detects ECAPs using as little battery power as possible.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed before the priority date of each claim of this application.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

In this specification, a statement that an element may be "at least one of" a list of options is to be understood to mean that the element may be any one of the listed options, or may be any combination of two or more of the listed options.

### SUMMARY OF THE INVENTION

Disclosed herein is a closed-loop neuromodulation device using a multiphasic stimulus cycle, which modulates the supply voltage from cycle to cycle, to a value proportional to the stimulus current in each cycle. This reduces the wasted energy (energy not delivered to tissue) as a proportion of the total delivered energy. The energy saving is greatest when the voltage waveforms on the stimulus and return electrodes are symmetrical about half the supply voltage over the full stimulus cycle. If such symmetry is not naturally present, the tissue voltage may be modulated between phases to impose such symmetry and thereby allow per-cycle supply voltage modulation to achieve the greatest efficiency. Any increase in artefact arising from the tissue voltage modulation may be dealt with through use of a triphasic stimulus cycle and / or other artefact reduction methods.

The device may additionally, or alternatively, modulate the supply voltage level from phase to phase to save power. In variable-current multiphasic stimulus, the first phase of the multiphasic stimulus pulse may comprise a lower amplitude "pre-charge pulse". This allows the supply voltage to be reduced during that phase. If the parameters of the pre-charge pulse are carefully chosen, artefact will also be significantly reduced. Artefact reduction methods may also be used if such parameter choice is limited, for example if the variable-current stimulus pulse is biphasic.

According to a first aspect of the present technology, there is provided an implantable neural stimulation device, the device comprising:
an electrode array comprising a plurality of electrodes, the electrodes comprising a first stimulus electrode and a second stimulus electrode;
a pulse generator connectable to the stimulus electrodes, the pulse generator configured to generate a multiphasic stimulus pulse of current from a supply voltage and deliver the multiphasic stimulus pulse via the stimulus electrodes to an electrically excitable tissue in order to evoke a neural response on a neural pathway in the electrically excitable tissue; and
modulation circuitry connectable to a regulation electrode of the plurality of electrodes, the modulation circuitry configured to modulate a voltage on the regulation electrode during the delivery of the multiphasic stimulus pulse such that a corresponding voltage on each stimulus electrode varies substantially symmetrically around a value which is about half the supply voltage over the multiphasic stimulus pulse.

In some embodiments, the implantable device further comprises a controller configured to adjust the supply voltage before the pulse generator generates the next multiphasic stimulus pulse.

According to a second aspect of the present technology, there is provided a method of stimulating electrically excitable tissue, the method comprising:
delivering a multiphasic stimulus pulse of current from a supply voltage via two stimulus electrodes of a plurality of electrodes to the electrically excitable tissue in order to evoke a neural response on a neural pathway in the electrically excitable tissue; and
modulating, with modulation circuitry, a voltage on a regulation electrode of the plurality of electrodes during the multiphasic stimulus pulse such that a corresponding voltage on each stimulus electrode varies symmetrically around a value which is about half the supply voltage over the multiphasic stimulus pulse.

According to a third aspect of the present technology, there is provided an implantable neural stimulation device comprising:
an electrode array comprising a plurality of electrodes, the electrodes comprising a first stimulus electrode and a second stimulus electrode;
a pulse generator connectable to the stimulus electrodes, the pulse generator configured to generate a variable-current multiphasic stimulus pulse from a supply voltage and deliver the variable-current multiphasic stimulus pulse via the stimulus electrodes to an electrically excitable tissue in order to evoke a neural response on a neural pathway in the electrically excitable tissue,
wherein an amplitude of a first phase of the variable-current multiphasic stimulus pulse is less than an amplitude of a second phase by a scaling factor;
regulation circuitry connectable to a regulation electrode of the plurality of electrodes, the regulation circuitry configured to regulate a voltage on the tissue in communication with the regulation electrode during the delivery of the variable-current multiphasic stimulus pulse; and
a controller configured to modulate the supply voltage during the delivery of the variable-current multiphasic stimulus pulse such that the supply voltage during the first phase is less than the supply voltage during the second phase.

In some embodiments, the regulation circuitry is configured to modulate a voltage on the regulation electrode during the variable-current multiphasic stimulus pulse such that a voltage on each stimulus electrode varies symmetrically around half the supply voltage over the variable-current multiphasic stimulus pulse.

In some embodiments, the controller is configured to adjust the supply voltage of the second phase before the pulse generator generates the next variable-current multiphasic stimulus pulse.

According to a fourth aspect of the present technology, there is provided a method of stimulating electrically excitable tissue, the method comprising:
delivering a variable-current multiphasic stimulus pulse of current from a supply voltage via two stimulus electrodes of a plurality of electrodes to the electrically excitable tissue in order to evoke a neural response on a neural pathway in the electrically excitable tissue;
wherein an amplitude of a first phase of the variable-current multiphasic stimulus pulse is less than an amplitude of a second phase by a scaling factor;
regulating a voltage on the tissue via a regulation electrode during the delivery of the variable-current multiphasic stimulus pulse; and
modulating the supply voltage during the delivery of the variable-current multiphasic stimulus pulse such that the supply voltage during the first phase is less than the supply voltage during the second phase.

In some embodiments of the first to fourth aspects of the invention, the voltage on each stimulus electrode varies symmetrically around a value which is about half the supply voltage over the multiphasic stimulus pulse.

In some embodiments of the first to fourth aspects of the invention, the voltage on each stimulus electrode varies substantially symmetrically about a value which is between 40 and 60% of the supply voltage over the multiphasic stimulus pulse, more preferably between 45% and 55% of the supply voltage over the multiphasic stimulus pulse, more preferably between 48% and 52% of the supply voltage over the multiphasic stimulus pulse, or most preferably 50% of the supply voltage over the multiphasic stimulus pulse.

In some embodiments of the first to fourth aspects of the invention, the modulation circuitry comprises a feedback amplifier with an output connected to the regulation electrode, a first input connected to a value which is about half the supply voltage, and a second input connected to a node connecting the stimulus electrodes. For example the regulation electrode may be one of the stimulus electrodes.

Some embodiments of the first to fourth aspects of the invention may further comprise measurement circuitry comprising a measurement amplifier, the measurement circuitry being configured to process a signal sensed at a first sense electrode and a second sense electrode of the plurality of electrodes subsequent to the delivered multiphasic stimulus pulse. The measurement circuitry may comprise one or more shields around respective leads to the measurement amplifier. The modulation circuitry may comprise a feedback amplifier with an output connected to the regulation electrode, a first input connected to a value which is about half the supply voltage, and a second input connected to a node connecting the stimulus electrodes; and the one or more shields may be driven by the feedback amplifier. Or, the one or more shields may be driven by a tissue-connected electrode of the plurality of electrodes via a buffer. Or, the one or more shields may be driven by a digital-to-analog-converted control signal.

Some embodiments of the first to fourth aspects of the invention may further comprise a controller. The modulation circuitry may comprise a digital-to-analog converter connected to the regulation electrode, the digital-to-analog converter being controlled by the controller. The regulation electrode may be one of the stimulus electrodes. The controller may be configured to adjust the supply voltage before the pulse generator generates a subsequent multiphasic stimulus pulse. The controller may be configured to adjust the supply voltage to at least an amplitude of the subsequent multiphasic stimulus pulse multiplied by a sum of tissue resistances at the stimulus electrodes. The controller may be configured to adjust the supply voltage using a digital-to-analog converter. The controller may be configured to adjust the supply voltage by controlling a switched-mode power supply.

In some embodiments of the first to fourth aspects of the invention the multiphasic stimulus pulse is triphasic. The modulation circuitry may be configured to modulate the voltage on the regulation electrode in between phases of the triphasic stimulus pulse to half the supply voltage.

In some embodiments of the third and fourth aspects of the invention the variable-current multiphasic stimulus pulse is a variable-current triphasic stimulus pulse, such that a charge delivered during the first phase is less than, and of opposite sign to, a charge delivered during the second phase, by a charge fraction. In such embodiments the charge fraction of the variable-current triphasic stimulus pulse may be chosen to minimise artefact. The regulation circuitry may be configured to modulate the voltage on the regulation electrode during the delivery of the variable-current multiphasic stimulus pulse such that a voltage on each stimulus electrode varies substantially symmetrically around a value which is about half the supply voltage over the variable-current multiphasic stimulus pulse. The controller may be configured to modulate the supply voltage during the delivery of the variable-current multiphasic stimulus pulse such that the supply voltage during the first phase is equal to the supply voltage during the second phase scaled by the scaling factor. The controller may be configured to adjust the supply voltage of the second phase before the pulse generator generates a subsequent variable-current multiphasic stimulus pulse.

Another aspect of the invention comprises an implantable device for evoking and measuring a neural response to stimulus, the device comprising: an electrode array comprising a plurality of electrodes, the electrodes comprising a first stimulus electrode and a second stimulus electrode, a first sense electrode and a second sense electrode; a pulse generator connectable to the stimulus electrodes, the pulse generator configured to generate a multiphasic stimulus pulse of current from a supply voltage via the stimulus electrodes to an electrically excitable tissue in order to evoke a neural response on a neural pathway in the electrically excitable tissue; modulation circuitry connectable to a regulation electrode of the plurality of electrodes, the modulation circuitry configured to modulate a voltage on the regulation electrode during the multiphasic stimulus pulse such that a voltage on each stimulus electrode varies symmetrically around half the supply voltage over the multiphasic stimulus pulse; and measurement circuitry, comprising a measurement amplifier, configured to measure the neural response evoked by the multiphasic stimulus pulse and sensed at the first sense electrode and the second sense electrode.

A further aspect of the invention provides a method of evoking and measuring a neural response to stimulus, the method comprising: delivering a multiphasic stimulus pulse of current from a supply voltage via two stimulus electrodes of a plurality of electrodes to an electrically excitable tissue in order to evoke a neural response on a neural pathway in the electrically excitable tissue; modulating, with modulation circuitry, a voltage on a regulation electrode of the plurality of electrodes during the multiphasic stimulus pulse such that a voltage on each stimulus electrode varies symmetrically around half the supply voltage over the multiphasic stimulus pulse; and measuring, with measurement circuitry comprising a measurement amplifier, the neural response evoked by the multiphasic stimulus pulse and sensed at a first sense electrode and a second sense electrode.

A still further aspect of the invention provides an implantable device for evoking and measuring a neural response to stimulus, the device comprising: an electrode array comprising a plurality of electrodes, the electrodes comprising a first stimulus electrode and a second stimulus electrode, a first sense electrode and a second sense electrode; a pulse generator connectable to the stimulus electrodes, the pulse generator configured to generate a variable-current multiphasic stimulus pulse from a supply voltage via the stimulus electrodes to an electrically excitable tissue in order to evoke a neural response on a neural pathway in the electrically excitable tissue, wherein an amplitude of a first phase of the variable-current multiphasic stimulus pulse is less than an amplitude of a second phase by a scaling factor; regulation circuitry connectable to a regulation electrode of the plurality of electrodes, the regulation circuitry configured to regulate a voltage on the tissue in communication with the regulation electrode during the variable-current multiphasic stimulus pulse; a controller configured to modulate the supply voltage during the variable-current multiphasic stimulus pulse such that the supply voltage during the first phase is less than the supply voltage during the second phase; and measurement circuitry configured to measure the neural response evoked by the variable-current multiphasic stimulus pulse and sensed at the first sense electrode and the second sense electrode.

Yet another aspect of the present invention provides a method of evoking and measuring a neural response to stimulus, the method comprising: delivering a variable-current multiphasic stimulus pulse of current from a supply voltage via two stimulus electrodes to an electrically excitable tissue in order to evoke a neural response on a neural pathway in the electrically excitable tissue; wherein an amplitude of a first phase of the variable-current multiphasic stimulus pulse is less than an amplitude of a second phase by a scaling factor; regulate a voltage on the tissue via a regulation electrode during the variable-current multiphasic stimulus pulse; modulating the supply voltage during the variable-current multiphasic stimulus pulse such that the supply voltage during the first phase is less than the supply voltage during the second phase; and measuring, with measurement circuitry, the neural response evoked by the variable-current multiphasic stimulus pulse and sensed at a first sense electrode and a second sense electrode.

References herein to estimation, determination, comparison and the like are to be understood as referring to an automated process carried out on data by a processor operating to execute a predefined procedure suitable to effect the described estimation, determination and/or comparison step(s). The technology disclosed herein may be implemented in hardware (e.g., using digital signal processors, application specific integrated circuits (ASICs) or field programmable gate arrays (FPGAs)), or in software (e.g., using instructions tangibly stored on non-transitory computer-readable media for causing a data processing system to perform the steps described herein), or in a combination of hardware and software. The disclosed technology can also be embodied as computer-readable code on a computer-readable medium. The computer-readable medium can include any data storage device that can store data which can thereafter be read by a computer system. Examples of the computer-readable medium include read-only memory ("ROM"), random-access memory ("RAM"), magnetic tape, optical data storage devices, flash storage devices, or any other suitable storage devices. The computer-readable medium can also be distributed over network-coupled computer systems so that the computer-readable code is stored and/or executed in a distributed fashion.

### BRIEF DESCRIPTION OF THE DRAWINGS

One or more implementations of the invention will now be described with reference to the accompanying drawings, in which:
Fig. 1 schematically illustrates an implanted spinal cord stimulator, according to one implementation of the present technology;
Fig. 2 is a block diagram of the stimulator of Fig. 1;
Fig. 3 is a schematic illustrating interaction of the implanted stimulator of Fig. 1 with a nerve;
Fig. 4 illustrates the typical form of an electrically evoked compound action potential (ECAP) of a healthy subject;
Fig. 5 is a schematic illustrating elements and inputs of a closed-loop neural stimulation system, according to one implementation of the present technology;
Fig. 6 shows a stimulus circuit model comprising a current source supplying a current from the supply or compliance voltage.
Fig. 7 shows the voltages on the stimulus and return electrodes in the stimulus circuit model of Fig. 6 over a biphasic stimulus cycle.
Fig. 8 shows the voltages on the stimulus and return electrodes in the stimulus circuit model of Fig. 6 over a biphasic stimulus cycle according to one implementation of the present technology.
Fig. 9 illustrates a circuit configured to implement the tissue voltage modulation to achieve the waveforms of Fig. 8 according to one implementation of the present technology.
Fig. 10 illustrates a circuit configured to implement the tissue voltage modulation to achieve the waveforms of Fig. 8 according to one implementation of the present technology.
Fig. 11 illustrates a circuit configured to implement the tissue voltage modulation to achieve the waveforms of Fig. 8 according to one implementation of the present technology.
Fig. 12 illustrates a circuit configured to implement the tissue voltage modulation to achieve the waveforms of Fig. 8 according to one implementation of the present technology.
Fig. 13 illustrates a circuit configured to implement the tissue voltage modulation to achieve the waveforms of Fig. 8 according to one implementation of the present technology.
Fig. 14 shows the voltages on the stimulus and return electrodes in the stimulus circuit model of Fig. 6 over a triphasic stimulus cycle.
Fig. 15 shows the voltages on the stimulus and return electrodes in the stimulus circuit model of Fig. 6 over a triphasic stimulus cycle according to one implementation of the present technology.
Fig. 16 illustrates a variable-current multiphasic stimulus waveform.
Fig. 17 is a graph illustrating a variety of variable-current triphasic pulses with different parameter values.
Fig. 18 is a graph illustrating portions of the artefact waveforms obtained from simulations using the variable-current triphasic waveforms of Fig. 17.
Fig. 19 illustrates a waveform of voltage delivered to tissue and a waveform of supply voltage according to one implementation of the present technology.

### DETAILED DESCRIPTION OF THE PRESENT TECHNOLOGY

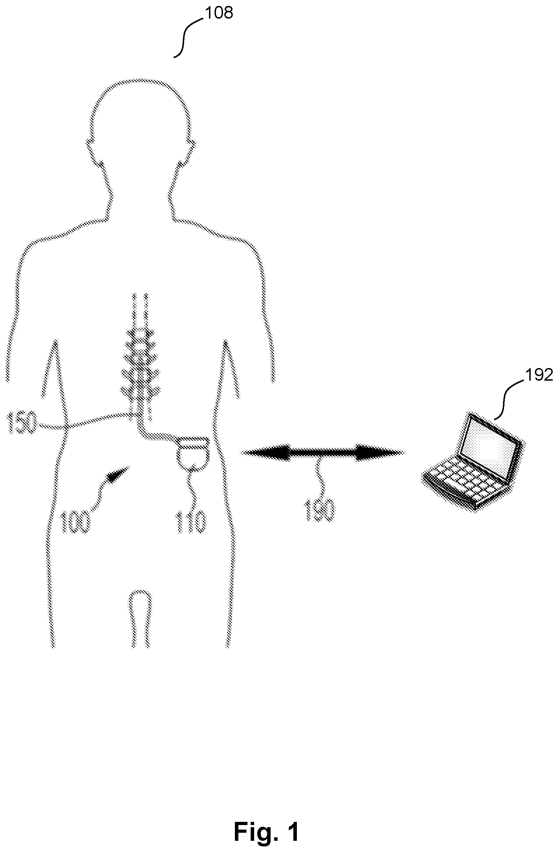

Fig. 1 schematically illustrates an implanted spinal cord stimulator 100 in a patient 108, according to one implementation of the present technology. Stimulator 100 comprises an electronics module 110 implanted at a suitable location. In one implementation, stimulator 100 is implanted in the patient's lower abdominal area or posterior superior gluteal region. In other implementations, the electronics module 110 is implanted in other locations, such as in a flank or sub-clavicularly. Stimulator 100 further comprises an electrode array 150 implanted within the epidural space and connected to the module 110 by a suitable lead. The electrode array 150 may comprise one or more electrodes such as electrode pads on a paddle lead, circular (e.g., ring) electrodes surrounding the body of the lead, conformable electrodes, cuff electrodes, segmented electrodes, or any other type of electrodes capable of forming unipolar, bipolar or multipolar electrode configurations for stimulation and measurement. The electrodes may pierce or affix directly to the tissue itself.

Numerous aspects of the operation of implanted stimulator 100 may be programmable by an external computing device 192, which may be operable by a user such as a clinician or the patient 108. Moreover, implanted stimulator 100 serves a data gathering role, with gathered data being communicated to external device 192 via a transcutaneous communications channel 190. Communications channel 190 may be active on a substantially continuous basis, at periodic intervals, at non-periodic intervals, or upon request from the external device 192. External device 192 may thus provide a clinical interface configured to program the implanted stimulator 100 and recover data stored on the implanted stimulator 100. This configuration is achieved by program instructions collectively referred to as the Clinical Programming Application (CPA) and stored in an instruction memory of the clinical interface.

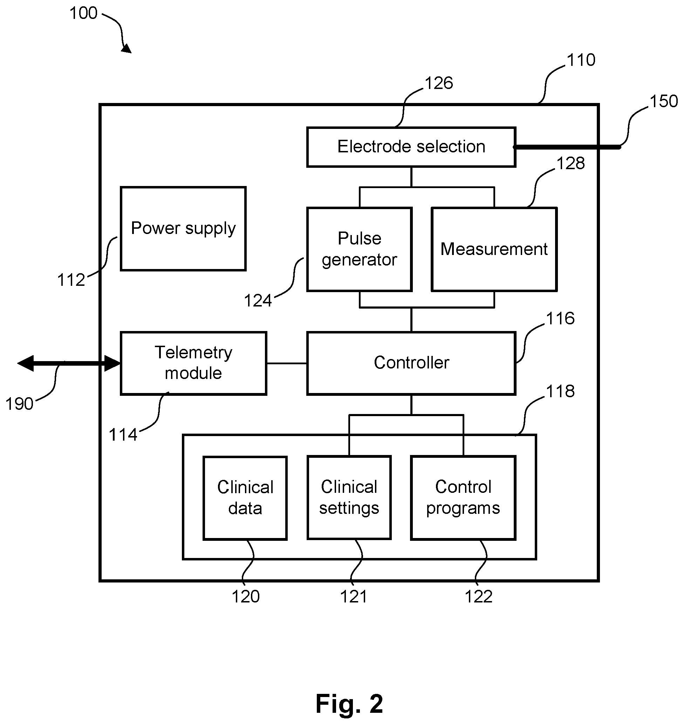

Fig. 2 is a block diagram of the stimulator 100. Electronics module 110 contains a battery 112 and a telemetry module 114. In implementations of the present technology, any suitable type of transcutaneous communications channel 190, such as infrared (IR), radiofrequency (RF), capacitive and/or inductive transfer, may be used by telemetry module 114 to transfer power and/or data to and from the electronics module 110 via communications channel 190. Module controller 116 has an associated memory 118 storing one or more of clinical data 120, clinical settings 121, control programs 122, and the like. Controller 116 controls a pulse generator 124 to generate stimuli, such as in the form of pulses, in accordance with the clinical settings 121 and control programs 122. Electrode selection module 126 switches the generated pulses to the selected electrode(s) of electrode array 150, for delivery of the pulses to the tissue surrounding the selected electrode(s). Measurement circuitry 128, which may comprise an amplifier and / or an analog-to-digital converter (ADC), is configured to process signals comprising neural responses sensed at measurement electrode(s) of the electrode array 150 as selected by electrode selection module 126.

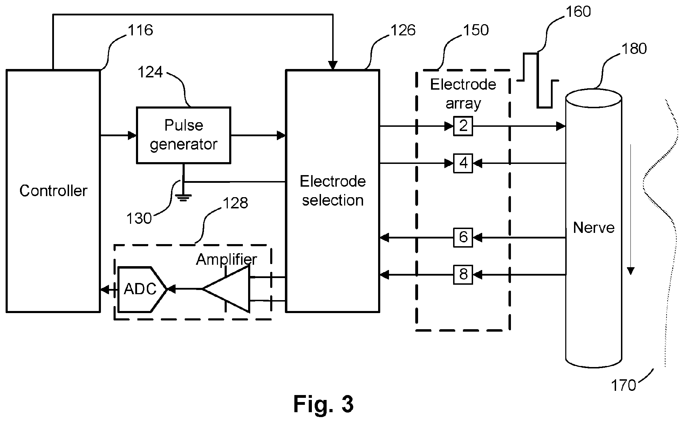

Fig. 3 is a schematic illustrating interaction of the implanted stimulator 100 with a nerve 180 in the patient 108. In the implementation illustrated in Fig. 3 the nerve 180 may be located in the spinal cord, however in alternative implementations the stimulator 100 may be positioned adjacent any desired neural tissue including a peripheral nerve, visceral nerve, parasympathetic nerve or a brain structure. Electrode selection module 126 selects a stimulus electrode 2 of electrode array 150 through which to deliver a pulse from the pulse generator 124 to surrounding tissue including nerve 180. A pulse may comprise one or more phases, e.g. a biphasic stimulus pulse (or cycle) 160 comprises two phases. A pulse comprising more than one phase is referred to as a multiphasic stimulus pulse. The electrode selection module 126 selects a stimulus electrode 2 to deliver the pulse to surrounding tissue including nerve 180. Electrode selection module 126 also selects a return electrode 4 of the electrode array 150 for stimulus charge recovery in each phase, to maintain a zero net charge transfer. Because a given electrode may act as both a stimulus and a return electrode over a complete multiphasic stimulus pulse, both electrodes are generally referred to as stimulus electrodes. The use of two electrodes in this manner for delivering and recovering current in each stimulus phase is referred to as bipolar stimulation. Alternative embodiments may apply other forms of bipolar stimulation, or may use a greater number of stimulus electrodes. Electrode selection module 126 is illustrated as connecting to a ground 130 of the pulse generator 124 to enable stimulus charge recovery via the return electrode 4. However, other connections for charge recovery may be used in other implementations.

Delivery of an appropriate stimulus from stimulus electrodes 2 and 4 to the nerve 180 evokes a neural response 170 comprising an evoked compound action potential (ECAP) which will propagate along the nerve 180 as illustrated. The ECAP may be evoked for therapeutic purposes, which in the case of a spinal cord stimulator for chronic pain may be to create paraesthesia at a desired location. To this end, the stimulus electrodes 2 and 4 are used to deliver stimuli periodically at any therapeutically suitable frequency, for example 30 Hz, although other frequencies may be used including frequencies as high as the kHz range. In alternative implementations, stimuli may be delivered in a non-periodic manner such as in bursts, or sporadically, as appropriate for the patient 108. To "fit" the stimulator 100 to the patient 108, a clinician may cause the stimulator 100 to deliver stimuli of various configurations which seek to produce a sensation that is experienced by the user as paraesthesia. When a stimulus configuration is found which evokes paraesthesia in a location and of a size which is congruent with the area of the patient's body affected by pain, the clinician nominates that configuration for ongoing use.

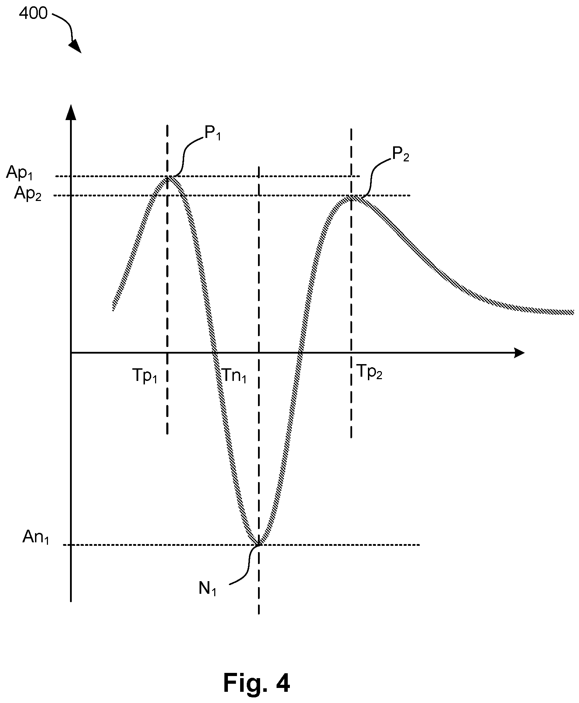

Fig. 4 illustrates the typical form of an ECAP 400 of a healthy subject, as recorded at a single measurement electrode referenced to the system ground 130. The shape and duration of the ECAP 400 shown in Fig. 4 is predictable because it is a result of the ion currents produced by the ensemble of fibres depolarising and generating action potentials (APs) in response to stimulation. The evoked action potentials (EAPs) generated synchronously among a large number of fibres sum to form the ECAP 400. The propagation velocity of the AP on each fibre is determined largely by the diameter of that fibre. The ECAP 400 generated from the synchronous depolarisation of a group of similar fibres comprises a positive peak P1, then a negative peak N1, followed by a second positive peak P2. This shape is caused by the region of activation passing the measurement electrode as the action potentials propagate along the individual fibres.

The ECAP may be recorded differentially using two measurement or sense electrodes, as illustrated in Fig. 3. Depending on the polarity of recording, a differential ECAP may take an inverse form to that shown in Fig. 4, i.e. a form having two negative peaks N1 and N2, and one positive peak P1. Alternatively, depending on the distance between the two measurement electrodes, a differential ECAP may resemble the time derivative of the ECAP 400, or more generally the difference between the ECAP 400 and a time-delayed copy thereof.

The ECAP 400 may be parametrised by any suitable parameter(s) of which some are indicated in Fig. 4. The amplitude of the positive peak P1 is *Ap*₁ and occurs at time *Tp*₁. The amplitude of the positive peak P2 is *Ap*₂ and occurs at time *Tp*₂. The amplitude of the negative peak P1 is *An*₁ and occurs at time *Tn*₁. The peak-to-peak amplitude is *Ap*₁ + *An*₁. A recorded ECAP will typically have a maximum peak-to-peak amplitude in the range of microvolts and a duration of 2 to 3 ms.

The stimulator 100 is further configured to detect the existence and measure the intensity of ECAPs 170 propagating along nerve 180, whether such ECAPs are evoked by the stimulus from electrodes 2 and 4, or otherwise evoked. To this end, any electrodes of the array 150 may be selected by the electrode selection module 126 to serve as measurement electrode 6 and measurement reference electrode 8, whereby the electrode selection module 126 selectively connects the chosen electrodes to the inputs of the measurement circuitry 128. Thus, signals sensed by the measurement electrodes 6 and 8 are passed to the measurement circuitry 128, which may comprise an amplifier and an analog-to-digital converter (ADC). The measurement circuitry 128 for example may operate in accordance with the teachings of the above-mentioned International Patent Publication No. WO2012/155183.

Neural responses obtained from the measurement electrodes 6, 8 via measurement circuitry 128 are processed by an ECAP detector implemented within controller 116 to obtain information regarding the effect of the applied stimulus upon the nerve 180. In some implementations, neural responses are processed by the ECAP detector in a manner which extracts and stores one or more parameters from each response or group of responses. In one such implementation, the parameter comprises a peak-to-peak ECAP amplitude in microvolts (µV). For example, the neural responses may be processed by the ECAP detector to determine the peak-to-peak ECAP amplitude in accordance with the teachings of International Patent Publication No. WO 2015/074121, the contents of which are incorporated herein by reference. Alternative implementations of the ECAP detector may extract and store an alternative parameter from the response to be stored, or may extract and store two or more parameters from the response.

For effective and comfortable operation of an implantable neuromodulation device such as the stimulator 100, it is desirable to maintain stimulus intensity within a therapeutic range.

To keep the applied stimulus intensity within the therapeutic range as patient posture varies, in some implementations an implantable neuromodulation device such as the stimulator 100 may adjust the applied stimulus intensity based on a feedback variable that is determined from one or more extracted ECAP parameters. In one implementation, the device may adjust the stimulus intensity to maintain the extracted ECAP amplitude at a target response intensity. For example, the device may calculate an error between a target ECAP value and a measured ECAP amplitude, and adjust the applied stimulus intensity to reduce the error as much as possible, such as by adding the scaled error to the current stimulus intensity. A neuromodulation device that operates by adjusting the applied stimulus intensity based on an extracted ECAP parameter is said to be operating in closed-loop mode and will also be referred to as a closed-loop neural stimulus (CLNS) device. By adjusting the applied stimulus intensity to maintain the extracted ECAP amplitude at an appropriate target response intensity, a CLNS device will generally keep the stimulus intensity within the therapeutic range as patient posture varies.

A CLNS device comprises a stimulator that takes a stimulus intensity value and converts it into a neural stimulus comprising a sequence of electrical pulses according to a predefined stimulation pattern. The stimulation pattern is characterised by multiple parameters including stimulus intensity (amplitude), pulse width, number of phases, order of phases, number of stimulus electrode poles (two for bipolar, three for tripolar etc.), and stimulus rate or frequency. At least one of the stimulus parameters, for example the stimulus amplitude, is controlled by the feedback loop.

In an example CLNS system, a user (e.g. the patient or a clinician) sets a target neural response intensity, and the CLNS device performs proportional-integral-differential (PID) control. In some implementations, the differential contribution is disregarded and the CLNS device uses a first order integrating feedback loop. The stimulator produces stimulus in accordance with a stimulus intensity parameter, which evokes a neural response in the patient. The evoked neural response (e.g. an ECAP) is detected and its amplitude measured by the CLNS device and compared to the target neural response intensity.

The measured neural response amplitude, and its deviation from the target neural response intensity, is used by the feedback loop to determine possible adjustments to the stimulus intensity parameter to maintain the neural response at the target intensity. If the target intensity is properly chosen, the patient receives consistently comfortable and therapeutic stimulation through posture changes and other perturbations to the stimulus / response behaviour.

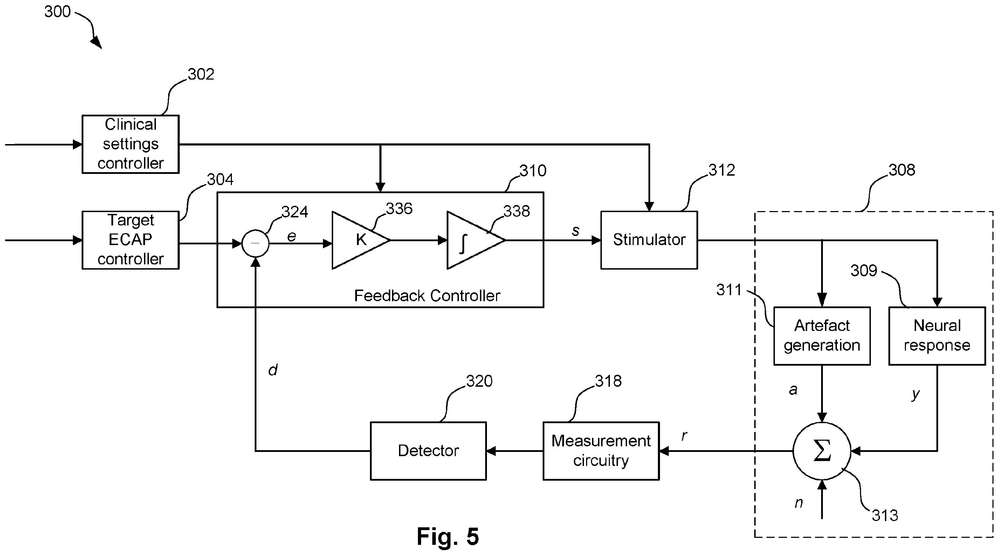

Fig. 5 is a schematic illustrating elements and inputs of a closed-loop neural stimulation (CLNS) system 300, according to one implementation of the present technology. The system 300 comprises a stimulator 312 which converts a stimulus intensity parameter (for example a stimulus current amplitude) *s*, in accordance with a set of predefined stimulus parameters, to a neural stimulus comprising a sequence of electrical pulses on the stimulus electrodes (not shown in Fig. 5). According to one implementation, the predefined stimulus parameters comprise the number and order of phases, the number of stimulus electrode poles, the pulse width, and the stimulus rate or frequency.

The generated stimulus crosses from the electrodes to the spinal cord, which is represented in Fig. 5 by the dashed box 308. The box 309 represents the evocation of a neural response *y* by the stimulus as described above. The box 311 represents the evocation of an artefact signal *a*, which is dependent on stimulus intensity and other stimulus parameters, as well as the electrical environment of the measurement electrode. Various sources of noise *n* may add to the evoked response *y* at the summing element 313 before the evoked response is measured, including electrical noise from external sources such as: 50 Hz mains power; electrical disturbances produced by the body such as neural responses evoked not by the device but by other causes such as peripheral sensory input; EEG; EMG; and electrical noise from measurement circuitry 318.

The neural recruitment arising from the stimulus is affected by mechanical changes, including posture changes, walking, breathing, heartbeat and so on. Mechanical changes may cause impedance changes, or changes in the location and orientation of the nerve fibres relative to the electrode array(s). As described above, the intensity of the evoked response provides a measure of the recruitment of the fibres being stimulated. In general, the more intense the stimulus, the more recruitment and the more intense the evoked response. An evoked response typically has a maximum amplitude in the range of microvolts, whereas the voltage resulting from the stimulus applied to evoke the response is typically several volts.

The total response signal *r* (including evoked neural response, artefact, and noise) is amplified by the measurement circuitry 318 and then measured by the ECAP detector 320. The ECAP detector 320 outputs a measured response intensity *d*. In one implementation, the neural response intensity comprises an ECAP value. The feedback controller 310 comprises a comparator 324 that compares the measured response intensity *d* to a target ECAP value to provide an indication of the difference between the measured response intensity *d* and the target ECAP value. This difference is the error value, *e*. The error value *e* is input into the feedback controller 310.

The feedback controller 310 calculates an adjusted stimulus intensity parameter, *s*, with the aim of maintaining a measured response intensity *d* equal to the target ECAP value. Accordingly, the feedback controller 310 adjusts the stimulus intensity parameter *s* to minimise the error value, *e*. In one implementation, the controller 310 utilises a first order integrating function, using a gain element 336 and an integrator 338, in order to provide suitable adjustment to the stimulus intensity parameter *s*. According to such an implementation, the current stimulus intensity parameter *s* may be computed by the feedback controller 310 as$s = \int Kedt$ where *K* is the gain of the gain element 336 (the controller gain). A target ECAP value is input to the feedback controller 310 via the target ECAP controller 304. In one embodiment, the target ECAP controller 304 provides an indication of a specific target ECAP value. In another embodiment, the target ECAP controller 304 provides an indication to increase or to decrease the present target ECAP value. The target ECAP controller 304 may comprise an input into the neural stimulus device, via which the patient or clinician can input a target ECAP value, or indication thereof. The target ECAP controller 304 may comprise memory in which the target ECAP value is stored, and from which the target ECAP value is provided to the feedback controller 310.

A clinical settings controller 302 provides clinical parameters to the system, including the gain *K* for the gain element 336 and the stimulation parameters for the stimulator 312. The clinical settings controller 302 may be configured to adjust the gain *K* of the gain element 336 to adapt the feedback loop to patient sensitivity. The clinical settings controller 302 may comprise an input into the neural stimulus device, via which the patient or clinician can adjust the clinical settings. The clinical settings controller 302 may comprise memory in which the clinical settings are stored, and are provided to components of the system 300.

In some implementations, two clocks (not shown) are used, being a stimulus clock operating at the stimulus frequency (e.g. 60 Hz) and a sample clock for sampling the measured response *r* (for example, operating at a sampling frequency of 10 kHz). As the ECAP detector 320 is linear, only the stimulus clock affects the dynamics of the CLNS system 300. On the next stimulus clock cycle, the stimulator 312 outputs a stimulus in accordance with the adjusted stimulus intensity *s*. Accordingly, there is a delay of one stimulus clock cycle before the stimulus intensity is updated in light of the error value *e*.

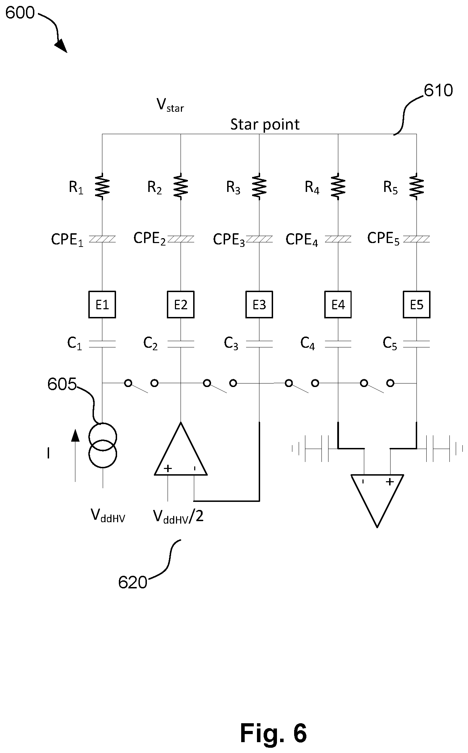

As mentioned above, power consumption is a prime consideration for implanted neuromodulation devices. On the other hand, it is desirable to hold the tissue voltage constant throughout a stimulus waveform so as to minimise stimulus artefact. Methods of holding the tissue voltage constant for singular and paired current sources are described by the present applicant in International Patent Publication Nos. WO2014/071445 and WO2014/071446 respectively, the contents of which are both incorporated herein by reference. The techniques disclosed in those publications are referred to here as Virtual Ground. Fig. 6 shows a stimulus circuit model 600 comprising a current source 605 supplying a current *I* from the supply or compliance voltage *V_{ddHV}.* The model 600 models the tissue being stimulated as a star-connected resistive network with each electrode Ei connected via a tissue resistance *Rᵢ* and continuous phase element CPEᵢ to a star point 610. In general, all the resistances *Rᵢ* are different. The *Cᵢ* are electrode capacitors. The model 600 also comprises a virtual ground arrangement 620 that drives the tissue voltage, modelled as the star point voltage *Vₛₜₐᵣ,* to a predetermined value that is, for example as illustrated, halfway between the supply voltage *V_{ddHV}* and the circuit ground. Switches in the circuit (not shown) switch the current source 605 between electrode E1 and electrode E2 in the multiple phases of a bipolar multiphasic stimulus pulse. The current *I* may therefore be identified with the stimulus intensity parameter s of Fig. 5. Further switches in the circuit (not shown) switch the virtual ground 620 between electrode E2 and electrode E1 in the multiple phases of a bipolar multiphasic stimulus pulse. Alternatively, electrode E2 may stay connected to the virtual ground 620 throughout the cycle and other switches (not shown) switch a current sink (not shown) so as to sink a current *I* to ground from electrode E1 during a phase of a multiphasic stimulus pulse.

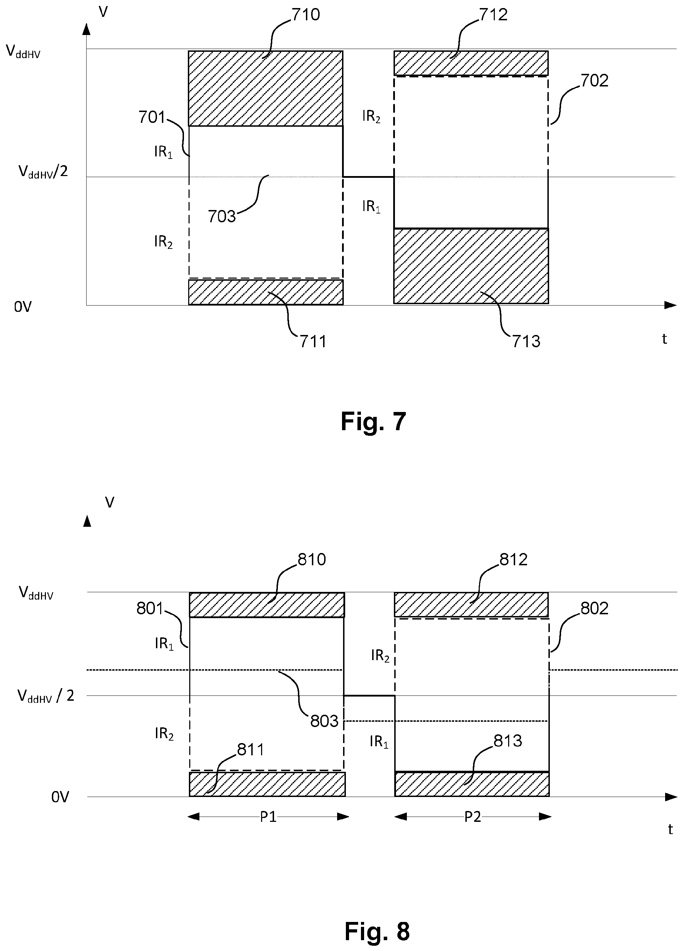

This arrangement results in wasted power in certain circumstances, as the waveforms in Fig. 7 illustrate. Fig. 7 shows the voltages on the stimulus and return electrodes E1 and E2 in the stimulus circuit model of Fig. 6 over a bipolar biphasic stimulus cycle as waveforms 701 (solid) and 702 (dashed), with the star point voltage *Vₛₜₐᵣ* 703 (dotted) being held constant at *V_{ddHV}* / 2 by a virtual ground arrangement. The waveform 701 on E1 oscillates between *Vₛₜₐᵣ + I*R*₁ and *Vₛₜₐᵣ - I*R*₁. The waveform 702 on E2 oscillates between *Vₛₜₐᵣ - I*R*₂ and *Vₛₜₐᵣ + I*R*₂*.* Two conditions must be met to avoid saturation of the current source(s):
- The supply voltage *V_{ddHV}* must be greater than the larger of *Vₛₜₐᵣ + I*R*₁ and *Vₛₜₐᵣ + I*R*₂*.*
- The star point voltage *Vₛₜₐᵣ* must be greater than the larger of *I*R*₁ and *I*R*₂*.*

It follows that the supply voltage *V_{ddHV}* must be greater than 2*I* times the larger of *R*₁ and *R*₂., i.e. *V_{ddHV}* > 2*I* max(*R*₁, *R*₂).

Since the current in each phase is constant, the energy is proportional to the voltage drop. The total energy supplied is proportional to the supply voltage *V_{ddHV},* while the energy actually delivered to the tissue is proportional to *I*(*R*₁+ *R*₂). The hatched areas 710, 711, 712, and 713 therefore represent energy supplied but not delivered to the tissue, i.e. wasted energy, which is nonzero because of the inequality above.

### Per-cycle supply voltage modulation

In a CLNS device, the stimulus amplitude *I* varies cycle by cycle. In one implementation, the controller 116, knowing the tissue resistances *R*₁ and *R*₂ of the stimulus electrodes and the stimulus amplitude *I* for the next cycle, may adjust the supply voltage *V_{ddHV}* before the next cycle such that ${V}_{ddHV} > 2 I max \left({R}_{1}, {R}_{2}\right)$ which will reduce all of the areas 710-713 and, in the limit, will eliminate either the areas 710 and 713 or the areas 711 and 712. If *R*₁ = *R*₂, all of areas 710-713 may be so eliminated, although in most cases *R*₁ ≠ *R*₂.

In another implementation, if the tissue resistances *R*₁ and *R*₂ are unknown, the controller 116 may monitor the stimulus electrode voltages using, for example, positive and negative peak detectors to identify the maximum excursion *Vₚₖ* of each stimulus electrode voltage above and below the tissue voltage *V_{ddHV}* / 2 during the current cycle. (Using the model of Fig. 6, *Vₚₖ* for electrode *i* is *IR*_{*i*.}) The controller may infer that during the next cycle, the maximum excursion *Vₚₖ* of each stimulus electrode will be scaled by the same ratio as the stimulus amplitude *I*. The controller 116 may then adjust the supply voltage *V_{ddHV}* before the next cycle such that *V_{ddHV}* is greater than twice the largest expected value of *Vₚₖ*.

If the electrode tissue resistances *R*₁ and *R*₂ are equal, per-cycle supply voltage modulation may be used to minimise power consumption by setting the supply voltage *V_{ddHV}* to slightly greater than 2**I***R*₁ for each cycle. However as long as *R*₁ is not equal to *R*₂ there will still be some wasted energy on account of the asymmetry of the electrode voltages between the phases.

### Tissue voltage modulation

In one implementation of the present technology, the star point (tissue) voltage *Vₛₜₐᵣ* may be modulated to a predetermined waveform over the stimulus pulse rather than held to a constant value by the virtual ground arrangement 620 of Fig. 6. In either case, however, whether the tissue voltage is being modulated or held constant, the tissue voltage is referred to herein as being regulated. Fig. 8 illustrates the resulting waveforms in one implementation. In this implementation, the star point voltage waveform 803 (dotted) oscillates between two levels, $\frac{{V}_{ddHV}}{2} + \frac{I}{2} \left({R}_{2}-{R}_{1}\right)$ during the first phase P1 and $\frac{{V}_{ddHV}}{2} - \frac{I}{2} \left({R}_{2}-{R}_{1}\right)$ during the second phase P2. The result is that the electrode voltage waveforms 801 (electrode E1, solid line) and 802 (electrode E2, dashed line) have equal and opposite excursions of $\frac{I}{2} \left({R}_{2}+{R}_{1}\right)$ about the level *V_{ddHV}* / 2 during each phase. In other words, the voltage on electrode E2 is equal and opposite to the voltage on electrode E1 referred to the level *V_{ddHV}* / 2 throughout each phase of the stimulus cycle. In yet other words, the voltages on electrodes E1 and E2 vary symmetrically about the level *V_{ddHV}* / 2 over the stimulus cycle.

This symmetry resulting from the modulation of the tissue voltage allows the supply voltage *V_{ddHV}* to be reduced below the above mentioned lower limit of 2*I* max(*R*₁, *R*₂). The amount of wasted energy is therefore reduced, as may be seen by comparing the hatched areas 810, 811, 812, and 813 in Fig. 8 to the hatched areas 710, 711, 712, and 713 in Fig. 7. In the limit, when the supply voltage is reduced to *I*(*R*₁+*R*₂), the hatched areas 810, 811, 812, and 813 may shrink to zero without saturating the current source(s), and this is true even when *R*₁ ≠ *R*₂, as is common. Tissue voltage modulation therefore removes the asymmetry resulting from imbalance of tissue resistances and allows wasted energy to be reduced (via per-cycle supply voltage modulation) as low as if the tissue resistances were balanced.

If the electrode tissue resistances *R*₁ and *R*₂ are equal, tissue voltage modulation yields no benefit compared to virtual ground, since if virtual ground drives the tissue voltage to *V_{ddHV}* / 2, the electrode voltage waveforms are symmetrical about *V_{ddHV}* / 2. Without tissue voltage modulation, there would be no need for the artefact-reducing measures described below.

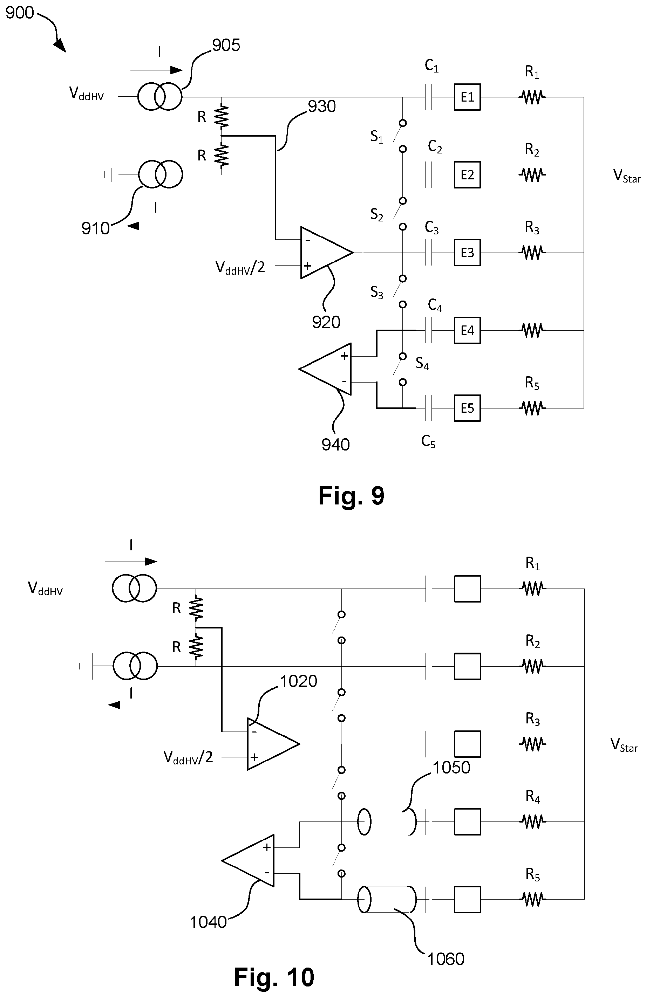

Fig. 9 illustrates a circuit 900 configured to implement the tissue voltage modulation to achieve the waveforms of Fig. 8 according to one implementation of the present technology. The virtual ground arrangement is not present. Instead, a current sink 910 is shown connected to E2 via an electrode capacitor C₂. Switches, e.g. configured in an H-bridge (not shown), switch the current source 905 and the current sink 910 between electrodes E1 and E2 over the two phases of a biphasic stimulus pulse. Two equal resistors labelled *R* in series connect the electrodes E1 and E2 via a node 930. A feedback amplifier 920 drives the star point via a regulation electrode, illustrated as E3, such that the node 930 is always at *V_{ddHV}* / 2 throughout the stimulus cycle. This ensures that the electrode voltages on E1 and E2 vary symmetrically about *V_{ddHV}* / 2 over the stimulus cycle, thereby achieving the tissue voltage modulation of Fig. 8. The amplifier 940 is the measurement amplifier connected to sense electrodes E4 and E5.

Modulating the tissue voltage as in Fig. 8, e.g. using the circuit 900, will tend to worsen artefact. It is therefore beneficial to include ways of reducing artefact when implementing tissue voltage modulation as in Fig. 8. Fig. 10 illustrates a circuit 1000 that is similar to the circuit 900, with like numbers indicating like components, except with the addition of shields 1050 and 1060 around the leads to the measurement amplifier 1040. The shields 1050 and 1060 are driven by the feedback amplifier 1020 to the star point voltage *Vₛₜₐᵣ*. This will tend to reduce the amount of artefact that reaches the measurement amplifier input leads as a result of the tissue voltage modulation. This and other methods of artefact minimisation are described in International Patent Application no. PCT/AU2022/050347 by the present applicant, the entire contents of which are hereby incorporated by reference.

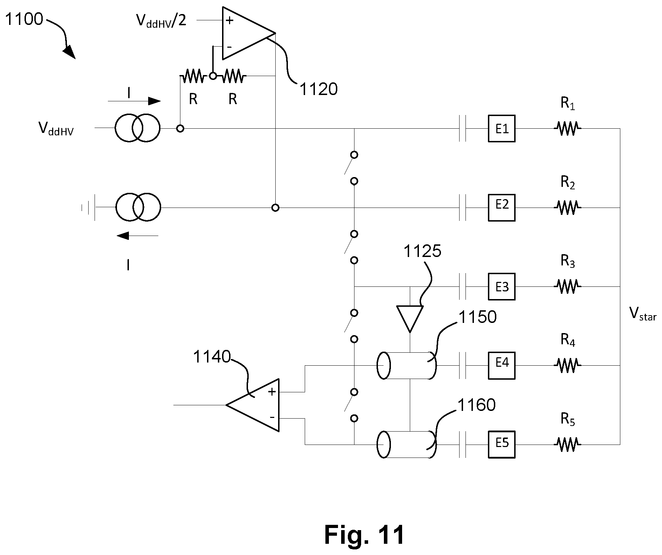

Fig. 11 illustrates a circuit 1100 that is similar to the circuit 1000, with like numbers indicating like components, except that the feedback amplifier 1120 is configured to drive the stimulus electrode E2, also acting as a regulation electrode, to a voltage equal and opposite the voltage on E1 throughout the stimulus cycle, relative to *V_{ddHV}* / 2. The shields 1150 and 1160 around the leads to the measurement amplifier 1140 are driven by a buffer 1125 connected to a further electrode E3. The circuit 1100 therefore achieves the tissue voltage modulation of Fig. 8.

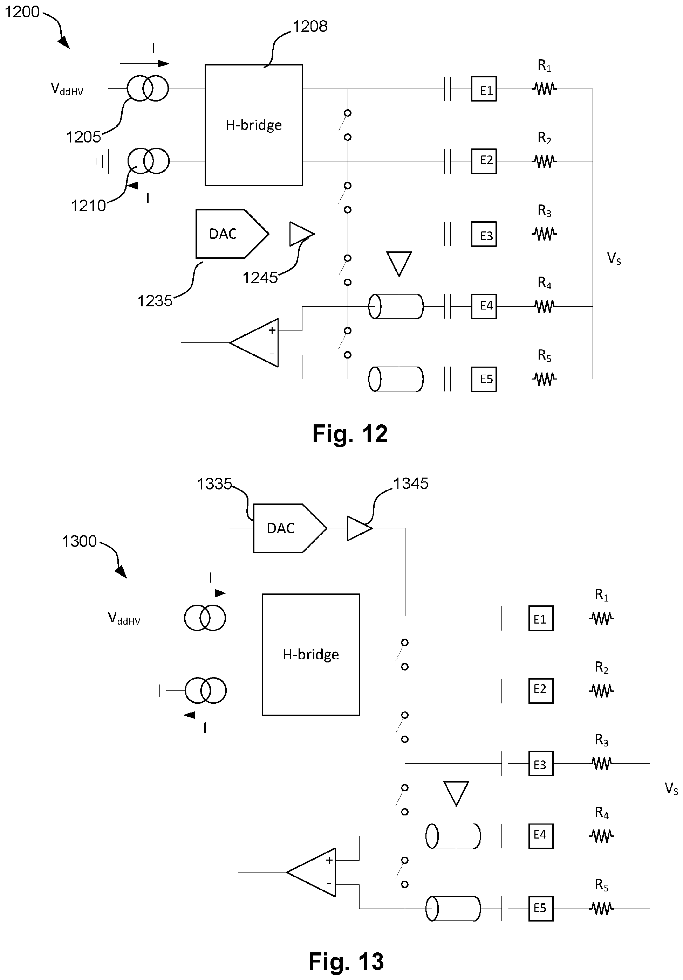

Fig. 12 illustrates a circuit 1200 that is similar to the circuit 1100, with like numbers indicating like components, except without the amplifier 1120. The circuit 1200 instead comprises a digital-to-analog converter (DAC) 1235, followed by an optional buffer 1245, that drives the tissue via E3 to a controllable voltage. The circuit 1200 therefore achieves the tissue voltage modulation of Fig. 8 if the output of the DAC 1235 is appropriately controlled by the controller 116. The circuit 1200 also contains an H-bridge 1208 that is used to switch the current sources 1205 and 1210 to the electrodes as described above. An H-bridge 1208 may be used in an analogous location and for the same purpose in any or all of the circuits 900, 1000, and 1100.

Fig. 13 illustrates a circuit 1300 that is similar to the circuit 1200, with like numbers indicating like components, except that the DAC 1335 and buffer 1345 drive the tissue via the stimulus electrode E1. The circuit 1300 therefore achieves the tissue voltage modulation of Fig. 8 if the output of the DAC 1335 is appropriately controlled by the controller 116. The controller 116 may set the voltage waveform to be output by the DAC 1235 or 1335 based on the stimulation current amplitude *I* computed by the feedback controller 310 for the next stimulus cycle.

The controller 116 may also modulate the supply voltage *V_{ddHV}* cycle by cycle based on the stimulation current amplitude *I* computed by the feedback controller 310 for the next stimulus cycle as described above. In one example, the controller 116 may set the supply voltage *V_{ddHV}* to the minimum value of *I*(*R*₁+*R*₂) plus a small safety margin. The controller 116 may use a DAC followed by a buffer, similar to the DAC-buffer pair 1235 / 1245, to set the supply voltage *V_{ddHV}* for each stimulus cycle in accordance with per-cycle supply voltage modulation. In another implementation, the controller 116 may control a switched-mode power supply to set the supply voltage *V_{ddHV}* for each stimulus cycle, either directly or via a DAC such as the DAC 1235.

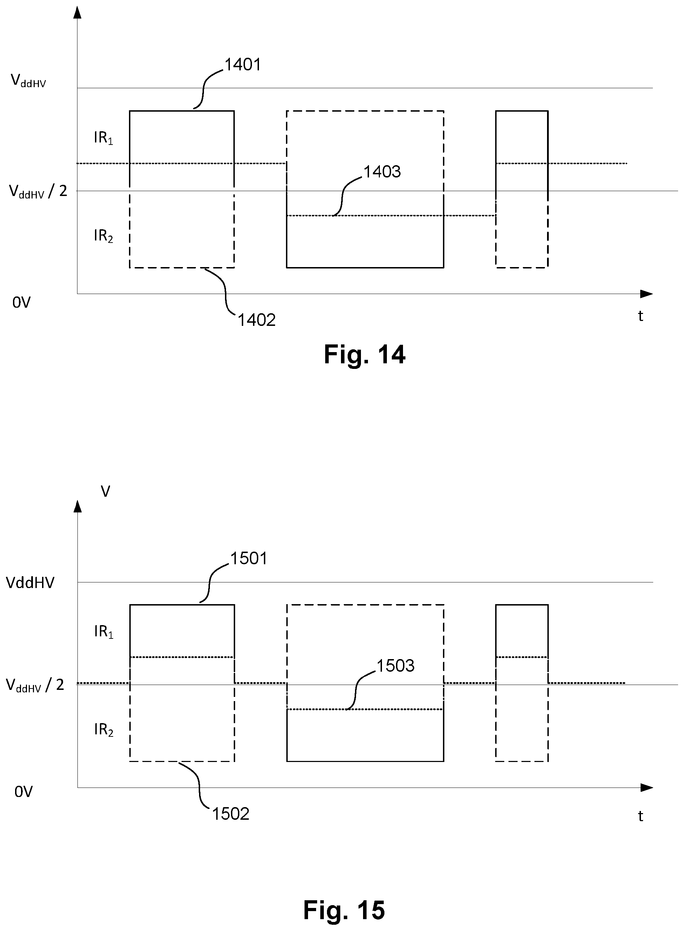

The disclosed implementations of Figs. 9 to 13 may also be used when the stimulus cycle is triphasic. Fig. 14 shows the voltages on the stimulus and return electrodes E1 and E2 in the stimulus circuit model of Fig. 6 over a triphasic stimulus cycle as waveforms 1401 (solid) and 1402 (dashed). The star point voltage *Vₛₜₐᵣ* 1403 (dotted) is modulated as in Fig. 8 to symmetrise the electrode voltage excursions around *V_{ddHV}* / 2 throughout the three phases of the triphasic cycle and thereby allow the supply voltage *V_{ddHV}* to be decreased to the minimum value of *I*(*R*₁+*R*₂), to save power. In between stimulus phases, the star point voltage *Vₛₜₐᵣ* 1403 does not change from its value at the end of the preceding phase, as the current sources are disabled, and the start point "floats", such that its value is maintained by stray capacitance. The star point voltage *Vₛₜₐᵣ* 1403 therefore consists of a single rectangular pulse per cycle, which will generate artefact as previously mentioned.

This artefact would be reduced if the star point voltage *Vₛₜₐᵣ* itself had a triphasic shape. Fig. 15 shows the voltages on the stimulus and return electrodes E1 and E2 in the stimulus circuit model of Fig. 6 over a triphasic stimulus cycle as waveforms 1501 (solid) and 1502 (dashed) according to one implementation of the present technology. The star point voltage *Vₛₜₐᵣ* 1503 (dotted) is modulated by any of the implementations in Figs. 12 to 13. The difference from Fig. 14 is that in between phases, during the first interphase gap and during the second interphase gap, the star point voltage *Vₛₜₐᵣ* 1503 is driven to *V_{ddHV}* / 2. The star point voltage waveform 1503 therefore has a triphasic shape with three distinct, balanced pulses in the three phases of the cycle, thereby reducing artefact. The implementations in Figs. 12 to 13 implementing the waveforms of Fig. 15 therefore minimise power consumption while achieving low artefact.

### Per-phase supply voltage modulation

The anodic phases of a multiphasic stimulus pulse, such as a triphasic stimulus pulse as illustrated in Fig. 14, do not generally stimulate neural tissue. As disclosed in International patent publication no. WO2017/219096 by the present applicant, the contents of which are incorporated herein by reference, the third phase of stimuli following that teaching typically comprises approximately one third (around 30-40%) of the charge delivered during the second phase, but opposite in sign to it, to reduce the amount of artefact, while the first phase typically comprises the remaining two thirds (or around 70-60%) of the second phase charge, also opposite in sign to it, for charge balance. The charge fraction represented by the first phase (e.g. two thirds in the example just described) is labelled α.

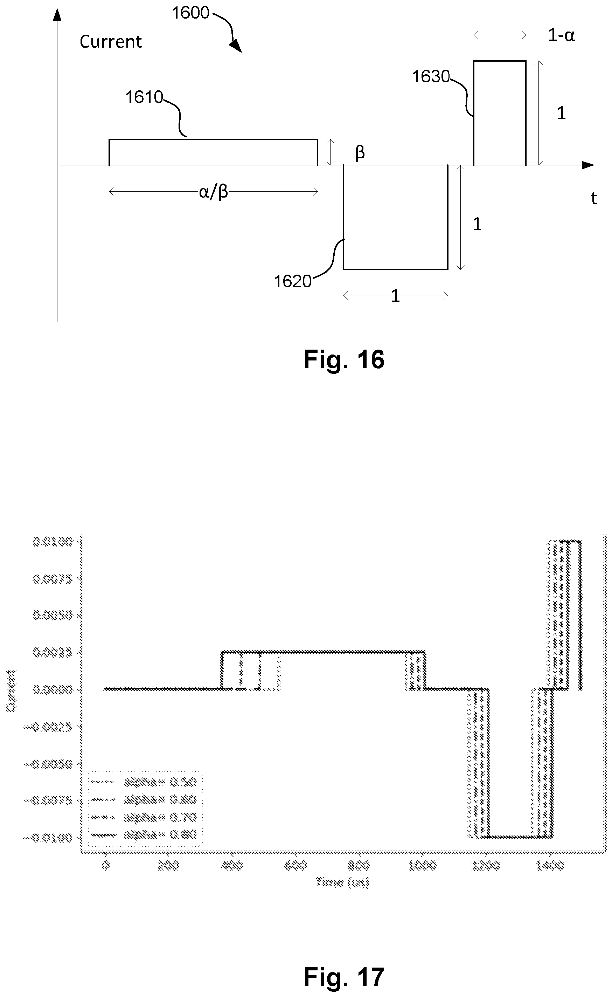

Fig. 16 illustrates a variable-current multiphasic stimulus waveform 1600 in which the charge (area) of the first phase 1610 is equal to α, while that of the third phase 1630 is equal to 1-α, thereby balancing the (negative) charge of the second phase 1620 (which is shown as unity). However, the amplitude of the first phase 1610 is reduced by a nonzero scaling factor β compared to the amplitude of the second phase 1620. The width (duration) of the first phase 1610 is correspondingly scaled by 1/β so that its charge remains equal to α. The factor β allows the amplitude and width of the first phase 1610 to vary in inverse proportion while maintaining the delivered charge of the first phase. A value of β equal to 1 yields a regular triphasic pulse of constant amplitude, as disclosed in the above-mentioned International patent publication no. WO2017/219096. Also, if α = 1, the variable-current multiphasic pulse 1600 is biphasic.

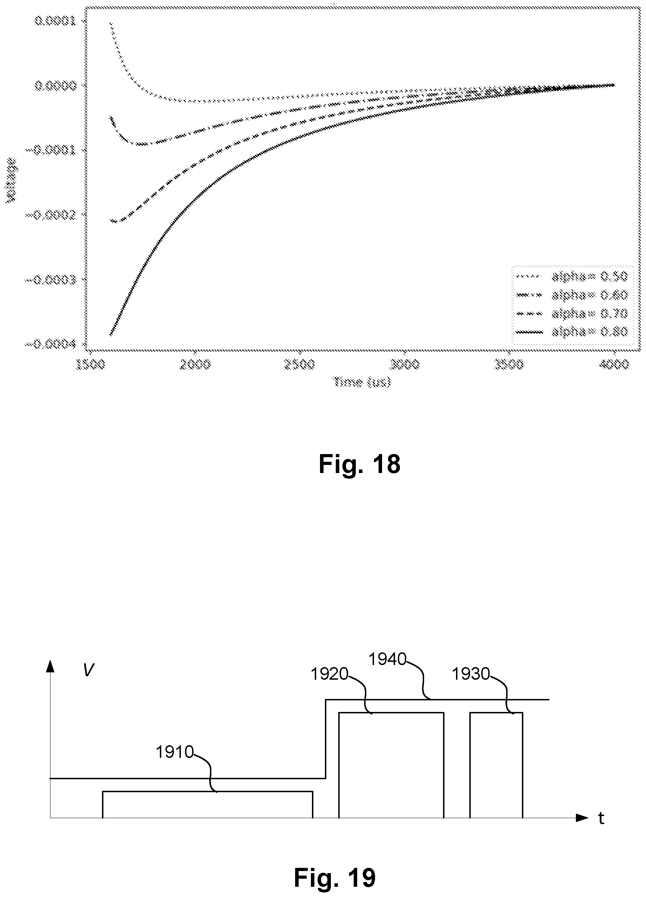

Because the first phase 1610 is anodic, choice of the scaling factor β does not affect the stimulus efficacy. Likewise, the first phase 1610 does not have much impact on the artefact generated by the pulse 1600. Fig. 17 is a graph illustrating a variety of variable-current triphasic pulses with the scaling factor β set to 0.25 and the charge fraction α varying between 0.5 and 0.8. Fig. 18 is a graph illustrating portions of the artefact waveforms obtained from simulations using the variable-current triphasic waveforms of Fig. 17. The graph shows the artefact switching from positive (when α = 0.5) to negative (when α = 0.8), having a minimum when α is between 0.6 and 0.7. A variable-current triphasic pulse with the correct choice of α and β is therefore potentially very low in artefact.

As mentioned above, the supply voltage *V_{ddHV}* needs to be above a minimum value at all times to avoid saturating the current sources. As described above, when the tissue voltage is regulated to a predetermined fixed value or waveform, rather than allowed to float, the minimum supply voltage is dependent on the stimulus current at any time. The current during the first phase of a variable-current multiphasic pulse is in general less than the current during the final phase by the scaling factor of β. Therefore, the supply voltage *V_{ddHV}* does not need to be at the same level during the first phase as during the final phase of a variable-current multiphasic pulse. The use of a variable-current multiphasic pulse therefore allows the supply voltage *V_{ddHV}* to be modulated within a stimulus cycle in order to save power. This is referred to herein as per-phase supply voltage modulation. In particular, the supply voltage *V_{ddHV}* during the first phase may be reduced by up to the scaling factor β compared to its value during the final phase, as illustrated in Fig. 19. The pulses 1910, 1920, and 1930 represent the voltages delivered to the tissue during the three phases of a variable-current triphasic pulse, when either the tissue resistances *R*₁ and *R*₂ are balanced, or tissue voltage modulation is used as described above. The waveform 1940 represents the supply voltage *V_{ddHV}* modulated within the stimulus cycle to be just greater than equal to its minimum value for each phase. As with per-cycle supply voltage modulation, the controller 116 may utilise a DAC followed by a buffer, similar to the DAC-buffer pair 1235 / 1245 in Fig. 12, to set the supply voltage *V_{ddHV}* for each phase of the stimulus cycle and thereby implement per-phase supply voltage modulation.

If used in conjunction with per-cycle supply voltage modulation as described above, along with either virtual ground if tissue resistances *R*₁ and *R*₂ are equal, or tissue voltage modulation if the tissue resistances *R*₁ and *R*₂ are unequal, per-phase supply voltage modulation allows the supply voltage *V_{ddHV}* during the second and third pulses 1920 and 1930 to be equal, and during the pulse 1910 to be as low as β times its value during the second and third pulses 1920 and 1930.

To estimate the power saving in this optimal case, the energy E required to generate the variable-current triphasic pulse with supply voltage *V_{ddHV}* held constant is 2 * *Q * V_{ddHV},* where *Q* is the charge delivered during the second phase 1920. Summing over the three phases, the energy *E'* required to generate the variable-current triphasic pulse with supply voltage *V_{ddHV}* modulated over the stimulus cycle as in Fig. 19 is (2-α+αβ) ** Q * V_{ddHV}.* The proportional energy saving may therefore be computed as $\frac{E - E ′}{E} = 1 - \frac{\left(2-α+αβ\right)}{2}$ which for β = 0.25 and α = 0.65 (to minimise artefact) evaluates to 24%.

If the tissue resistances *R*₁ and *R*₂ are unbalanced and tissue voltage modulation and per-cycle supply voltage modulation are not used in conjunction with the per-phase supply voltage modulation of Fig. 19, the required supply voltage to avoid saturation will be higher and the power saving will be reduced from the optimal value estimated above. However, the power saving of per-phase supply voltage modulation used alone will still be greater than zero. Additionally, the artefact generated by variable-current triphasic stimulation will be even lower without tissue voltage modulation.

Per-phase supply voltage modulation may also be used with variable-current biphasic stimulation (when α is equal to 1). As in variable-current triphasic, the supply voltage *V_{ddHV}* during the first phase may be reduced by a factor of up to β compared to its value during the final phase. The power saving will be greater (for example, equal to 37.5% when β = 0.25) than with variable-current triphasic stimulation. However, the artefact will be greater than in variable-current triphasic, particularly if tissue voltage modulation is used. The artefact reduction methods described above may be implemented to reduce this effect.

It will be appreciated by persons skilled in the art that numerous variations and/or modifications may be made to the invention as shown in the specific embodiments without departing from the spirit or scope of the invention as broadly described.

For example, while embodiments have been described in which the tissue voltage is modulated to oscillate around a predetermined value that is halfway between the supply voltage *V_{ddHV}* and the circuit ground, alternative embodiments of the present invention may instead select the predetermined value to differ somewhat from precisely the midpoint between the supply voltage *V_{ddHV}* and the circuit ground, such as being 40% or 60% of the supply voltage value relative to ground. In addition, or alternatively, the amplitude of the oscillation may be other than $\frac{I}{2} \left({R}_{2}-{R}_{1}\right)$, as described in relation to Fig. 8. For example, the embodiment of Fig. 8 may be modified such that the star point voltage waveform is regulated to oscillate or alternate between, $\frac{{V}_{ddHV}}{2} + \frac{I}{n} \left({R}_{2}-{R}_{1}\right)$ during the first phase P1 and $\frac{{V}_{ddHV}}{2} - \frac{I}{n} \left({R}_{2}-{R}_{1}\right)$ during the second phase P2, where *n* > 2. By setting *n* > 2, rather than *n* = 2 as described in relation to Fig. 8, or by setting the predetermined value to be greater or lesser than the midpoint between the supply voltage *V_{ddHV}* and the circuit ground, such alternative embodiments of the present invention provide for a reduction in asymmetry of the stimulus electrode voltages relative to the supply midpoint, without seeking precise symmetry of those waveforms about the midpoint, should this be more appropriate in some circumstances. In other embodiments, the predetermined value may be a midpoint, or close to a midpoint, between a positive supply rail and a negative supply rail.

As a further example, while embodiments have been described that are suitable for bipolar stimulation, alternative embodiments are suitable for multipolar stimulation. In tripolar stimulation, for example, a central stimulus electrode Eᵢᵢ is interposed between two return electrodes Eᵢ and Eᵢᵢᵢ, , as distinct from a bipolar arrangement having a stimulus electrode E1 and a single return electrode E2. The voltages on the two return electrodes Eᵢ and Eᵢᵢᵢ are substantially equal throughout the stimulus cycle. The voltage waveform on the two return electrodes Eᵢ and ᵢᵢᵢ are of similar character to the voltage waveform on the single return electrode E2 illustrated in the bipolar arrangement of Fig. 7 (with virtual ground in place), except that the resistance *R*₂ of the bipolar arrangement should be replaced by the resistance of *R*ᵢ||*R*ᵢᵢᵢ(*R*ᵢ in parallel with *R*ᵢᵢᵢ) in this tripolar arrangement. To obtain symmetry of the electrode voltage waveforms between the two phases of a biphasic waveform delivered in a tripolar manner, the tissue voltage may therefore be modulated according to the same waveform as previously described in relation to Fig. 8, except that the resistance *R*₂ from that bipolar arrangement may be replaced by the resistance *R*ᵢ||*R*ᵢᵢᵢ in this tripolar arrangement. In the limit, the supply voltage may therefore be reduced to *I*(*R*ᵢᵢ+*R*ᵢ||*R*ᵢᵢᵢ) to eliminate the hatched areas of wasted power. Similar embodiments may be applied to triphasic tripolar stimulation as illustrated in Figs. 14 and 15.

The present embodiments are, therefore, to be considered in all respects as illustrative and not limiting or restrictive.

### LABEL LIST

| | |
|---|---|
| stimulator | 100 |
| patient | 108 |
| module | 110 |
| battery | 112 |
| telemetry module | 114 |
| controller | 116 |
| memory | 118 |
| clinical data | 120 |
| clinical settings | 121 |
| control program | 122 |
| pulse generator | 124 |
| electrode selection module | 126 |
| measurement circuitry | 128 |
| system ground | 130 |
| electrode array | 150 |
| stimulus cycle | 160 |
| ECAP | 170 |
| nerve | 180 |
| transcutaneous communications channel | 190 |
| external device | 192 |
| CLNS system | 300 |
| clinical settings controller | 302 |
| target ECAP controller | 304 |
| box | 308 |
| box | 309 |
| feedback controller | 310 |
| box | 311 |
| stimulator | 312 |
| element | 313 |
| measurement circuitry | 318 |
| ECAP detector | 320 |
| comparator | 324 |
| gain element | 336 |
| integrator | 338 |
| ECAP | 400 |
| circuit model | 600 |
| current source | 605 |
| star point | 610 |
| virtual ground arrangement | 620 |
| waveform | 701 |
| waveform | 702 |
| star point voltage waveform | 703 |
| area | 710 |
| area | 711 |
| area | 712 |
| area | 713 |
| electrode voltage waveform | 801 |
| star point voltage waveform | 803 |
| area | 810 |
| area | 811 |
| area | 812 |
| area | 813 |
| circuit | 900 |
| current source | 905 |
| current sink | 910 |
| amplifier | 920 |
| node | 930 |
| amplifier | 940 |
| circuit | 1000 |
| amplifier | 1020 |
| measurement amplifier | 1040 |
| shield | 1050 |
| shield | 1060 |
| circuit | 1100 |
| amplifier | 1120 |
| Buffer | 1125 |
| Amplifier | 1140 |
| Shield | 1150 |
| Shield | 1160 |
| circuit | 1200 |
| current source | 1205 |
| H - bridge | 1208 |
| current source | 1210 |
| DAC | 1235 |
| buffer | 1245 |
| circuit | 1300 |
| DAC | 1335 |
| buffer | 1345 |
| waveform | 1401 |
| star point voltage | 1403 |
| waveform | 1501 |
| star point voltage | 1503 |
| pulse | 1600 |
| first phase | 1610 |
| second phase | 1620 |
| pulse | 1910 |
| second pulse | 1920 |
| third pulse | 1930 |
| waveform | 1940 |

### VARIOUS CLAUSES RELATED TO POTENTIAL ASPECTS OF THE INVENTION ARE NOW PRESENTED:

1. An implantable neural stimulation device, the device comprising:
   an electrode array comprising a plurality of electrodes, the electrodes comprising a first stimulus electrode and a second stimulus electrode;
   a pulse generator connectable to the stimulus electrodes, the pulse generator configured to generate a multiphasic stimulus pulse of current from a supply voltage and deliver the multiphasic stimulus pulse via the stimulus electrodes to an electrically excitable tissue in order to evoke a neural response on a neural pathway in the electrically excitable tissue; and
   modulation circuitry connectable to a regulation electrode of the plurality of electrodes, the modulation circuitry configured to modulate a voltage on the regulation electrode during the delivery of the multiphasic stimulus pulse such that a corresponding voltage on each stimulus electrode varies substantially symmetrically around a value which is about half the supply voltage over the multiphasic stimulus pulse.
2. The implantable device of clause 1, wherein the voltage on each stimulus electrode varies symmetrically around a value which is about half the supply voltage over the multiphasic stimulus pulse.
3. The implantable device of any one of clauses 1 to 2, wherein the voltage on each stimulus electrode varies substantially symmetrically about a value which is between 40 and 60% of the supply voltage over the multiphasic stimulus pulse.
4. The implantable device of clause 3, wherein the value is between 45% and 55% of the supply voltage over the multiphasic stimulus pulse.
5. The implantable device of clause 4, wherein the value is between 48% and 52% of the supply voltage over the multiphasic stimulus pulse.
6. The implantable device of clause 5, wherein the value is 50% of the supply voltage over the multiphasic stimulus pulse.
7. The implantable device of any one of clauses1 to 6, wherein the modulation circuitry comprises a feedback amplifier with an output connected to the regulation electrode, a first input connected to a value which is about half the supply voltage, and a second input connected to a node connecting the stimulus electrodes.
8. The implantable device of clause 7, wherein the regulation electrode is one of the stimulus electrodes.
9. The implantable device of any one of clauses 1 to 8, further comprising measurement circuitry comprising a measurement amplifier, the measurement circuitry being configured to process a signal sensed at a first sense electrode and a second sense electrode of the plurality of electrodes subsequent to the delivered multiphasic stimulus pulse.
10. The implantable device of clause 9, wherein the measurement circuitry comprises one or more shields around respective leads to the measurement amplifier.
11. The implantable device of clause 10, wherein:
   the modulation circuitry comprises a feedback amplifier with an output connected to the regulation electrode, a first input connected to a value which is about half the supply voltage, and a second input connected to a node connecting the stimulus electrodes; and
   the one or more shields are driven by the feedback amplifier.
12. The implantable device of clause 10, wherein the one or more shields are driven by a tissue-connected electrode of the plurality of electrodes via a buffer.
13. The implantable device of clause 10, wherein the one or more shields are driven by a digital-to-analog-converted control signal.
14. The implantable device of any one of clauses 1 to 13, further comprising a controller.
15. The implantable device of clause 14, wherein the modulation circuitry comprises a digital-to-analog converter connected to the regulation electrode, the digital-to-analog converter being controlled by the controller.
16. The implantable device of clause 15, wherein the regulation electrode is one of the stimulus electrodes.
17. The implantable device of any one of clauses 1 to 16, wherein the multiphasic stimulus pulse is triphasic.
18. The implantable device of clause 17, wherein the modulation circuitry is configured to modulate the voltage on the regulation electrode in between phases of the triphasic stimulus pulse to half the supply voltage.
19. The implantable device of any one of clauses 14 to 16, wherein the controller is configured to adjust the supply voltage before the pulse generator generates a subsequent multiphasic stimulus pulse.
20. The implantable device of clause 19, wherein the controller is configured to adjust the supply voltage to at least an amplitude of the subsequent multiphasic stimulus pulse multiplied by a sum of tissue resistances at the stimulus electrodes.
21. The implantable device of any one of clauses 19 to 20, wherein the controller is configured to adjust the supply voltage using a digital-to-analog converter.
22. The implantable device of any one of clauses 19 to 20, wherein the controller is configured to adjust the supply voltage by controlling a switched-mode power supply.
23. A method of stimulating electrically excitable tissue, the method comprising:
   delivering a multiphasic stimulus pulse of current from a supply voltage via two stimulus electrodes of a plurality of electrodes to the electrically excitable tissue in order to evoke a neural response on a neural pathway in the electrically excitable tissue; and
   modulating, with modulation circuitry, a voltage on a regulation electrode of the plurality of electrodes during the multiphasic stimulus pulse such that a corresponding voltage on each stimulus electrode varies symmetrically around a value which is about half the supply voltage over the multiphasic stimulus pulse.
24. The method of clause 23, wherein the voltage on each stimulus electrode varies symmetrically around a value which is about half the supply voltage over the multiphasic stimulus pulse.
25. The method of any one of clauses 23 to 24, wherein the voltage on each stimulus electrode varies substantially symmetrically about a value which is between 40 and 60% of the supply voltage over the multiphasic stimulus pulse.
26. The method of clause 25, wherein the value is between 45% and 55% of the supply voltage over the multiphasic stimulus pulse.
27. The method of clause 26, wherein the value is between 48% and 52% of the supply voltage over the multiphasic stimulus pulse.
28. The method of clause 27, wherein the value is 50% of the supply voltage over the multiphasic stimulus pulse.
29. The method of any one of clauses 23 to 28, wherein the modulation circuitry comprises a feedback amplifier with an output connected to the regulation electrode, a first input connected to a value which is about half the supply voltage, and a second input connected to a node connecting the stimulus electrodes.
30. The method of clause 29, wherein the regulation electrode is one of the stimulus electrodes.
31. The method of any one of clauses 23 to 30, further comprising processing, with measurement circuitry comprising a measurement amplifier, a signal sensed at a first sense electrode and a second sense electrode of the plurality of electrodes subsequent to the delivered multiphasic stimulus pulse.
32. The method of clause 31, wherein the measurement circuitry comprises one or more shields around respective leads to the measurement amplifier.
33. The method of clause 32, wherein the modulation circuitry comprises a feedback amplifier with an output connected to the regulation electrode, a first input connected to a value which is about half the supply voltage, and a second input connected to a node connecting the stimulus electrodes, further comprising driving, by the feedback amplifier, the one or more shields.
34. The method of clause 32, further comprising driving the one or more shields by a tissue-connected electrode of the plurality of electrodes via a buffer.
35. The method of clause 32, further comprising driving the one or more shields by a digital-to-analog-converted control signal.
36. The method of any one of clauses 23 to 35, wherein the modulation circuitry comprises a digital-to-analog converter connected to the regulation electrode.
37. The method of clause 36, wherein the regulation electrode is one of the stimulus electrodes.
38. The method of any one of clauses 23 to 37, wherein the multiphasic stimulus pulse is triphasic.
39. The method of clause 38, further comprising modulating, by the modulation circuitry, the voltage on the regulation electrode in between phases of the triphasic stimulus pulse to half the supply voltage.
40. The method of any one of clauses 23 to 39, further comprising adjusting the supply voltage before delivering a subsequent multiphasic stimulus pulse.
41. The method of clause 40, wherein the adjusting comprises adjusting the supply voltage to at least an amplitude of the next multiphasic stimulus pulse multiplied by the sum of the tissue resistances at the stimulus electrodes.
42. The method of any one of clauses 40 to 41, wherein adjusting the supply voltage uses a digital-to-analog converter.
43. The method of any one of clauses 40 to 41, wherein the adjusting the supply voltage comprises controlling a switched-mode power supply.
44. An implantable neural stimulation device comprising:
   an electrode array comprising a plurality of electrodes, the electrodes comprising a first stimulus electrode and a second stimulus electrode;
   a pulse generator connectable to the stimulus electrodes, the pulse generator configured to generate a variable-current multiphasic stimulus pulse from a supply voltage and deliver the variable-current multiphasic stimulus pulse via the stimulus electrodes to an electrically excitable tissue in order to evoke a neural response on a neural pathway in the electrically excitable tissue,
   wherein an amplitude of a first phase of the variable-current multiphasic stimulus pulse is less than an amplitude of a second phase by a scaling factor;
   regulation circuitry connectable to a regulation electrode of the plurality of electrodes, the regulation circuitry configured to regulate a voltage on the tissue in communication with the regulation electrode during the delivery of the variable-current multiphasic stimulus pulse; and
   a controller configured to modulate the supply voltage during the delivery of the variable-current multiphasic stimulus pulse such that the supply voltage during the first phase is less than the supply voltage during the second phase.
45. The implantable device of clause 44, wherein the controller is configured to modulate the supply voltage during the delivery of the variable-current multiphasic stimulus pulse using a digital-to-analog converter.
46. The implantable device of clause 44, wherein the controller is configured to modulate the supply voltage during the delivery of the variable-current multiphasic stimulus pulse by controlling a switched-mode power supply.
47. The implantable device of any one of clauses 44 to 46, wherein the variable-current multiphasic stimulus pulse is a variable-current triphasic stimulus pulse, such that a charge delivered during the first phase is less than, and of opposite sign to, a charge delivered during the second phase, by a charge fraction.
48. The implantable device of clause 47, wherein the charge fraction of the variable-current triphasic stimulus pulse is chosen to minimise artefact.
49. The implantable device of any one of clauses 44 to 48, wherein the regulation circuitry is configured to modulate the voltage on the regulation electrode during the delivery of the variable-current multiphasic stimulus pulse such that a voltage on each stimulus electrode varies substantially symmetrically around a value which is about half the supply voltage over the variable-current multiphasic stimulus pulse.
50. The implantable device of any one of clauses 44 to 49, wherein the controller is configured to modulate the supply voltage during the delivery of the variable-current multiphasic stimulus pulse such that the supply voltage during the first phase is equal to the supply voltage during the second phase scaled by the scaling factor.
51. The implantable device of any one of clauses 44 to 50, wherein the controller is configured to adjust the supply voltage of the second phase before the pulse generator generates a subsequent variable-current multiphasic stimulus pulse.
52. The implantable device of any one of clauses 44 to 51, further comprising measurement circuitry configured to process a signal sensed at a first sense electrode and a second sense electrode of the plurality of electrodes subsequent to the delivered variable-current multiphasic stimulus pulse.
53. A method of stimulating electrically excitable tissue, the method comprising:
   delivering a variable-current multiphasic stimulus pulse of current from a supply voltage via two stimulus electrodes of a plurality of electrodes to the electrically excitable tissue in order to evoke a neural response on a neural pathway in the electrically excitable tissue;
   wherein an amplitude of a first phase of the variable-current multiphasic stimulus pulse is less than an amplitude of a second phase by a scaling factor;
   regulating a voltage on the tissue via a regulation electrode during the delivery of the variable-current multiphasic stimulus pulse; and
   modulating the supply voltage during the delivery of the variable-current multiphasic stimulus pulse such that the supply voltage during the first phase is less than the supply voltage during the second phase.
54. The method of clause 43, wherein the modulating the supply voltage during the delivery of the variable-current multiphasic stimulus pulse uses a digital-to-analog converter.
55. The method of clause 43, wherein the modulating the supply voltage during the delivery of the variable-current multiphasic stimulus pulse comprises controlling a switched-mode power supply.
56. The method of any one of clauses 53 to 55, wherein the variable-current multiphasic stimulus pulse is a variable-current triphasic stimulus pulse, such that a charge delivered during the first phase is less than, and of opposite sign to, a charge delivered during the second phase, by a charge fraction.
57. The method of clause 56, further comprising choosing the charge fraction of the variable-current triphasic stimulus pulse to minimise artefact.
58. The method of any one of clauses 53 to 57, wherein the regulating the voltage on the tissue comprises modulating a voltage on the regulation electrode during the delivery of the variable-current multiphasic stimulus pulse such that a voltage on each stimulus electrode varies substantially symmetrically around a value which is about half the supply voltage over the variable-current multiphasic stimulus pulse.
59. The method of any one of clauses 53 to 58, further comprising adjusting the supply voltage of the second phase before delivering a subsequent variable-current multiphasic stimulus pulse.
60. The method of any one of clauses 53 to 59, wherein the modulating the supply voltage during the delivery of the variable-current multiphasic stimulus pulse comprises setting the supply voltage during the first phase to be equal to the supply voltage during the second phase scaled by the scaling factor.
61. The method of any one of clauses 53 to 60, further comprising processing, with measurement circuitry, a signal sensed subsequent to the delivery of the variable-current multiphasic stimulus pulse.

## Claims

1. An implantable neural stimulation device comprising:
an electrode array comprising a plurality of electrodes, the electrodes comprising a first stimulus electrode and a second stimulus electrode;
a pulse generator connectable to the stimulus electrodes, the pulse generator configured to generate a variable-current multiphasic stimulus pulse from a supply voltage and deliver the variable-current multiphasic stimulus pulse via the stimulus electrodes to an electrically excitable tissue in order to evoke a neural response on a neural pathway in the electrically excitable tissue,
wherein an amplitude of a first phase of the variable-current multiphasic stimulus pulse is less than an amplitude of a second phase by a scaling factor;
regulation circuitry connectable to a regulation electrode of the plurality of electrodes, the regulation circuitry configured to regulate a voltage on the tissue in communication with the regulation electrode during the delivery of the variable-current multiphasic stimulus pulse; and
a controller configured to modulate the supply voltage during the delivery of the variable-current multiphasic stimulus pulse such that the supply voltage during the first phase is less than the supply voltage during the second phase.

2. The implantable device of claim 1, wherein the controller is configured to modulate the supply voltage during the delivery of the variable-current multiphasic stimulus pulse using a digital-to-analog converter.

3. The implantable device of claim 1, wherein the controller is configured to modulate the supply voltage during the delivery of the variable-current multiphasic stimulus pulse by controlling a switched-mode power supply.

4. The implantable device of any one of claims 1 to 3, wherein the variable-current multiphasic stimulus pulse is a variable-current triphasic stimulus pulse, such that a charge delivered during the first phase is less than, and of opposite sign to, a charge delivered during the second phase, by a charge fraction.

5. The implantable device of claim 4, wherein the charge fraction of the variable-current triphasic stimulus pulse is chosen to minimise artefact.

6. The implantable device of any one of claims 1 to 5, wherein the regulation circuitry is configured to modulate the voltage on the regulation electrode during the delivery of the variable-current multiphasic stimulus pulse such that a voltage on each stimulus electrode varies substantially symmetrically around a value which is about half the supply voltage over the variable-current multiphasic stimulus pulse.

7. The implantable device of any one of claims 1 to 6, wherein the controller is configured to modulate the supply voltage during the delivery of the variable-current multiphasic stimulus pulse such that the supply voltage during the first phase is equal to the supply voltage during the second phase scaled by the scaling factor.

8. The implantable device of any one of claims 1 to 7, wherein the controller is configured to adjust the supply voltage of the second phase before the pulse generator generates a subsequent variable-current multiphasic stimulus pulse.

9. A method of stimulating electrically excitable tissue, the method comprising:
delivering a variable-current multiphasic stimulus pulse of current from a supply voltage via two stimulus electrodes of a plurality of electrodes to the electrically excitable tissue in order to evoke a neural response on a neural pathway in the electrically excitable tissue;
wherein an amplitude of a first phase of the variable-current multiphasic stimulus pulse is less than an amplitude of a second phase by a scaling factor;
regulating a voltage on the tissue via a regulation electrode during the delivery of the variable-current multiphasic stimulus pulse; and
modulating the supply voltage during the delivery of the variable-current multiphasic stimulus pulse such that the supply voltage during the first phase is less than the supply voltage during the second phase.

10. The method of claim 9, wherein the modulating the supply voltage during the delivery of the variable-current multiphasic stimulus pulse uses a digital-to-analog converter.

11. The method of claim 9, wherein the modulating the supply voltage during the delivery of the variable-current multiphasic stimulus pulse comprises controlling a switched-mode power supply.

12. The method of any one of claims 9 to 11, wherein the variable-current multiphasic stimulus pulse is a variable-current triphasic stimulus pulse, such that a charge delivered during the first phase is less than, and of opposite sign to, a charge delivered during the second phase, by a charge fraction.

13. The method of claim 12, further comprising choosing the charge fraction of the variable-current triphasic stimulus pulse to minimise artefact.

14. The method of any one of claims 9 to 13, wherein the regulating the voltage on the tissue comprises modulating a voltage on the regulation electrode during the delivery of the variable-current multiphasic stimulus pulse such that a voltage on each stimulus electrode varies substantially symmetrically around a value which is about half the supply voltage over the variable-current multiphasic stimulus pulse.

15. The method of any one of claims 9 to 14, further comprising adjusting the supply voltage of the second phase before delivering a subsequent variable-current multiphasic stimulus pulse.

16. The method of any one of claims 9 to 15, wherein the modulating the supply voltage during the delivery of the variable-current multiphasic stimulus pulse comprises setting the supply voltage during the first phase to be equal to the supply voltage during the second phase scaled by the scaling factor.
